# EUROPEAN PATENT SPECIFICATION

(11) **EP 1 183 270 B1**
(45) Date of publication and mention of the grant of the patent: **19.05.2010**
(21) Application number: 00937636.9
(22) Date of filing: 19.05.2000
(51) Int. Cl.: C07K 14/435, C07K 16/18, A61K 38/16, A61K 38/17, G01N 33/53, G01N 33/567, C12N 5/10, C12N 15/12

(54) **NOVEL ORGANIC ANION TRANSPORT PROTEINS**
TRANSPORTPROTEINE FUER ORGANISCHE ANIONEN
NOUVELLES PROTEINES DE TRANSFERT D'ANION ORGANIQUE

(30) Priority: 20.05.1999 US 135081 P
(43) Date of publication of application: 06.03.2002
(73) Proprietor: Bristol-Myers Squibb Company, Princeton NJ 08543-4000 (US)
(72) Inventor: Kirchgessner, Todd, G., Flemington, NJ 08822 (US); HSIANG, Bonnie, Trenton, NJ 08638 (US); ZHU, Yingjie, Killingworth, CT 06419 (US); WU, Yuli, Newtown, PA 18940 (US); WANG, Zhaoqing, Piscataway, NJ 08854 (US); LYNCH, Jean, S., Ringoes, NJ 08551 (US); HUANG, Xin, Cranbury, NJ 08512 (US); YANG, Wen-Pin, Princeton, NJ 08540 (US)
(74) Representative: Vossius & Partner
(86) International application number: PCT/US2000/013939
(87) International publication number: WO 2000/071566

(56) References cited:
- WO-A-95/17905
- WO-A-97/31111
- JACQUEMIN E ET AL: "EXPRESSION CLONING OF A RAT LIVER NA+-INDEPENDENT ORGANIC ANION TRANSPORTER" PROCEEDINGS OF THE NATIONAL ACADEMY OF SCIENCES OF USA, NATIONAL ACADEMY OF SCIENCE. WASHINGTON, US, vol. 91, January 1994 (1994-01), pages 133-137, XP002931742 ISSN: 0027-8424 -& DATABASE UniProt 1 November 1995 (1995-11-01), XP002326904 retrieved from EBI Database accession no. P46720 -& DATABASE EMBL 6 March 1994 (1994-03-06), XP002326905 retrieved from EBI Database accession no. L19031.1
- KULLAK-UBLICK ET AL: "molecular and functional characterization of an organic anion transporting polypeptide cloned from human liver" GASTROENTEROLOGY, SAUNDERS, PHILADELPHIA, PA,, US, vol. 109, October 1995 (1995-10), pages 1274-1282, XP002092892 ISSN: 0016-5085 -& DATABASE UniProt 1 November 1995 (1995-11-01), XP002326906 retrieved from EBI Database accession no. P46721 -& DATABASE EMBL 19 February 1996 (1996-02-19), XP002326907 retrieved from EBI Database accession no. U21943.1
- DATABASE EMBL 28 February 1995 (1995-02-28), XP002327003 retrieved from EBI Database accession no. HS48825
- JACQUEMIN ET AL.: 'Expression Cloning of a Rat Liver Na+-Independent Organic Anion Transporter' PROC. NATL. ACAD. SCI., USA vol. 91, January 1994, pages 133 - 137, XP002933087
- KIM, R.B.: EUR. J. CLINICAL INVESTIGATION, vol. 33, 2003, pages 1-5,
- MARZOLINI, C. ET AL.: PHARMACOGENOMICS, vol. 5, 2004, pages 273-282,

## Description

### Field of the Invention

The invention claims isolated nucleic acid encoding all or a portion of novel member of the organic anion transport protein ("OATP") designated OATP2.

Also claimed are vectors containing the nucleic acid sequences, host cells containing the vectors and polypeptides having all or part of the amino acid sequence of OATP2.

Tissue expression of the transporter is described as well as some of its substrates. Also claimed are uses for this novel OATP, including for targeting drugs to specific tissues, for modulating the concentration of endogenous substrates, and for identifying a substrate capable of being transported by the novel OATP of the invention.

### Background of the Invention

The liver functions in the clearance of a large variety of metabolic products, drugs and other xenobiotics by transporting them across the sinusoidal membrane into the hepatocyte. Several classes of transport systems have been described that mediate these processes including the Na+/taurocholate cotransporter polypeptide, NTCP, in rat and human liver (Hagenbuch, B., et al. (1991) Proc. Natl. Acad. Sci. USA 88:10629-33; Hagenbuch, B. et al., (1994) J. Clin. Invest. 93:1326-31) and a family of organic anion transporting polypeptides (OATPs) that are principally expressed in liver, kidney and brain, and transport a broad spectrum of substrates in a sodium-independent manner (Meier, P.J., et al., (1997) Hepatology 26:1667-77; Wolkoff, A.W., (1996) Semin. Liver Dis. 16:121-127). The distribution of this latter family of transporters in liver, kidney and choroid plexus in the brain is thought to reflect common physiological requirements of these organs for the clearance of a multitide of organic anions. There are three OATP isoforms in the rat: roatp1 (Jacquemin, E., et al., (1994) Proc. Natl. Acad. Sci. USA 91:133-37); roatp2 (Noe, B.A., et al., (1997) Proc. Natl. Acad. Sci. USA 94:10346-50; and roatp3 (Abe, T., et al., (1998) J. Biol. Chem. 273:11395-401). In addition to bile acids, OATPs are known to transport a variety of other compounds. These include, depending on the transporter, unconjugated and conjugated steroids such as estrone sulfate, estradiol-17B-glucuronide, aldosterone, and cardiac glycosides (Boussuyt, X., et al., (1996) J. Pharmacol. Exp. Ther. 276:891-6; Boussuyt, X. (1996) J. Hepatol. 25:733-8; Kanai, N., et al., (1996) Am. J. Physiol. 270:F319-F325; Kanai, N., et al., (1996) Am. J. Physiol. 270:F326-F331; Noe, B.A., et al., (1997) Proc. Natl. Acad Sci. USA 94:10346-50). Bromosulfophthalien (Jacquemin, E., et al., (1994) Proc. Natl. Acad. Sci. USA 91:133-7); mycotoxin (Kontaxi, M., et al., (1996) J. Pharmacol. Exp. Ther. 279:1507-13); leukotriene C₄ (Li, L., et al., (1998) J. Biol. Chem. 273:16184-91); and thyroid hormone (Abe, T., et al., (1998) J. Biol. Chem. 273:11395) are additional substrates.

Several proteins have been identified. Jacquemin,E., et al., (1994) Proc. Natl. Acad. Sci. U.S.A., 91:133-137 reported the first cloning and identification of a member of the OATP transporter family, namely the rat oatp1. The first cloning and identification of a human OATP was reported in Kullak-Ublick, G.A., et al., (1995) Gastroenterology, 109:1274-1282. Its expression was found in liver, kidney brain and other organs. The authors concluded, based on substrate specificities, that it was not the human orthologue of rat oatp1.

Substrate specificities of rat oatp1 are discussed in Kullak-Ublick, G.A. et al., (1994) Hepatology, 20:411-416, while substrate specificities of human OATP are discussed in Bossuyt, X., et al., (1996) J. Hepatol., 25:733-738.

Data was later discovered showing that rat oatp1 is involved in the transport of steroids (Bossuyt, X., et al., (1996) J. Pharmacol. Exp. Ther., 276:891-896), and that human OATP acts as a transporter for the psychoactive hormone DHEAS (Kullak-Ublick, G.A., et al., (1998) FEBS Lett., 424:173-176). For a review of the OATP family and organic anoin transport in the liver, see Wolkoff, A.W., (1996) Semin. LiverDis., 16:121-127*.*

A third rat OATP isoform that was shown to transport thyroid hormones T3 and T4 was cloned and reported in Abe,T., et al., (1998) J. Biol. Chem., 273:22395-22401.

### Summary of the Invention

The present invention encompasses a novel organic anion transport protein ("OATP") and polynucleotides encoding said OATP. The OATP disclosed herein is designated OATP2. A polynucleotide sequence of the OATP is disclosed herein, along with the deduced amino acid sequence. The cDNA encoding the OATP of the present invention has been deposited with the American Type Culture Collection and given Accession Numbers ATCC 207213 (OATP2).

The present inventors sequenced the cDNA encoding the novel OATP and determined the primary sequence of the deduced protein. Disclosed herein are the nucleic acid sequence (SEQ ID NO:1) and amino acid sequence (SEQ ID NO:2) of OATP2;

The OATP of the present invention can be produced by: (1) inserting the cDNA of a disclosed OATP into an appropriate expression vector; (2) transfecting the expression vector into an appropriate transfection host(s); (3) growing the transfected host(s) in appropriate culture media; and (4) assaying the transport activity in the transfected cells.

The present invention therefore provides a purified and isolated nucleic acid molecule, preferably a DNA molecule, having a sequence which codes for an OATP, or an oligonucleotide fragment of the nucleic acid molecule which is unique to an OATP of the invention. The purified and isolated nucleic acid molecule has the sequence as shown in SEQ ID NO:1 (OATP2).

The invention also contemplates a double stranded nucleic acid molecule comprising a nucleic acid molecule of the invention or an oligonucleotide fragment thereof hydrogen bonded to a complementary nucleotide base sequence.

The terms "isolated and purified nucleic acid", "isolated and purified polynucleotide", "substantially pure nucleic acid", and "substantially pure polynucleotide", e.g., substantially pure DNA, refer to a nucleic acid molecule which is one or both of the following: (1) not immediately contiguous with either one or both of the sequences, e.g., coding sequences, with which it is immediately contiguous (i.e., one at the 5' end and one at the 3'end) in the naturally occurring genome of the organism from which the nucleic acid is derived; or (2) which is substantially free of a nucleic acid sequence with which it occurs in the organism from which the nucleic acid is derived. The term includes, for example, a recombinant DNA which is incorporated into a vector, e.g., into an autonomously replicating plasmid or virus, or into the genomic DNA of a prokaryote or eukaryote, or which exists as a separate molecule (e.g., a cDNA or a genomic DNA fragment produced by PCR or restriction endonuclease treatment) independent of other DNA sequences. Substantially pure or isolated and purified DNA also includes a recombinant DNA which is part of a hybrid gene encoding additional OATP sequence.

Further described is (a) an isolated and purified nucleic acid molecule comprising a sequence encoding all or a portion of a protein having the amino acid sequence as shown in SEQ ID NO:2 (OATP2); (b) nucleic acid sequences complimentary to (a); (c) nucleic acid sequences which exhibit at least 80%, at least 90%, at least 95%, and at least 98% sequence identity to (a); or (d) a fragment of (a) or (b) that is at least 18 bases and which will hybridize to (a) or (b) under stringent conditions.

The degree of homology (percent sequence identity) between two sequences may be determined, for example, by comparing the two sequences using computer programs commonly employed for this purpose. One suitable program is the GAP computer program described by Devereux et al., (1984) Nucl. Acids Res. 12:387. The GAP program utilizes the alignment method of Needleman and Wunsch (1970) J. Mol. Biol. 48:433, as revised by Smith and Waterman (1981) Adv. Appl. Math. 2:482. Briefly, the GAP program defines percent identity as the number of aligned symbols (i.e., nucleotides or amino acids) which are identical, divided by the total number of symbols in the shorter of the two sequences.

As used herein the term "stringent conditions" encompasses conditions known in the art under which a nucleotide sequence will hybridize to: (a) an isolated and purified nucleic acid molecule comprising a sequence encoding a protein having the amino acid sequence as shown herein, or to (b) a nucleic acid sequence complementary to (a). Screening polynucleotides under stringent conditions may be carried out according to the method described in Nature, 313:402-404 (1985). Polynucleotide sequences capable of hybridizing under stringent conditions with the polynucleotides of the present invention may be, for example, allelic variants of the disclosed DNA sequences, or may be derived from other sources. General techniques of nucleic acid hybridization are disclosed by Sambrook et al., "Molecular Cloning: A Laboratory Manual", 2nd Ed., Cold Spring Harbor Laboratory, Cold Spring Harbor, New York (1984); and by Haymes et al., "Nucleic Acid Hybridization: A Practical Approach", IRL Press, Washington, D.C. (1985).

Further described are polynucleotides and amino acid sequences having one or more structural mutations including replacement, deletion or insertion mutations. For example, a signal peptide may be deleted, or conservative amino acid substitutions may be made to generate a protein that is still biologically competent or active.

The invention further contemplates a recombinant molecule comprising a nucleic acid molecule of the present invention or an oligonucleotide fragment thereof and an expression control sequence operatively linked to the nucleic acid molecule or oligonucleotide fragment. A transformant host cell including a recombinant molecule of the invention is also provided.

In another aspect, the invention features a cell or purified preparation of cells which include a novel gene encoding an OATP of the present invention, or which otherwise misexpresses a gene encoding an OATP of the present invention. The cell preparation can consist of human or non-human cells, e.g., rodent cells, e.g., mouse or rat cells, rabbit cells, non-human primate cells, or pig cells. In preferred embodiments, the cell or cells include an OATP transgene, e.g., a heterologous form of an OATP gene, e.g., a gene derived from humans (in the case of a non-human cell). The OATP transgene can be misexpressed, e.g., overexpressed or underexpressed. In other preferred embodiments, the cell or cells include a gene which misexpresses an endogenous OATP gene, e.g., a gene that expression of which is disrupted, e.g., a knockout. Such cells can serve as a model for studying disorders which are related to mutated or misexpressed OATP alleles for use in drug screening.

Still further, the invention provides plasmids which comprise the nucleic acid molecules of the invention. Also encompassed within the invention are vectors comprising the nucleic acid sequences disclosed herein, as well as host cells comprising said vectors.

The present invention also includes a novel OATP of the present invention, or an active part thereof. A biologically competent or active form of the protein or part thereof is also referred to herein as an "active OATP or part thereof".

The invention further contemplates antibodies having specificity against an epitope of an OATP of the present invention or part of the protein. These antibodies may be polyclonal or monoclonal. The antibodies may be labeled with a detectable substance and they may be used, for example, to detect a novel OATP of the invention in tissue and cells. Additionally, the antibodies of the present invention, or portions thereof, may be used to make targeted antibodies that destroy OATP expressing cells (e.g., antibody-toxin fusion proteins, or radiolabelled antibodies).

The invention also permits the construction of nucleotide probes which encode part or all of a novel OATP protein of the invention or a part of the protein. Thus, the invention also relates to a probe comprising a nucleotide sequence coding for a protein, which displays the properties of a novel OATP of the invention or a peptide unique to the protein. The probe may be labeled, for example, with a detectable (e.g., radioactive) substance and it may be used to select from a mixture of nucleotide sequences a nucleotide sequence coding for a protein which displays the properties of a novel OATP of the invention.

The present invention also provides a transgenic non-human animal (e.g., a rodent, e.g., a mouse or a rat, a rabbit or a pig) or embryo all of whose germ cells and somatic cells contain a recombinant molecule of the invention, preferably a recombinant molecule comprising a nucleic acid molecule of the present invention encoding an OATP of the invention or part thereof. The recombinant molecule may comprise a nucleic acid sequence encoding an OATP of the present invention with a structural mutation, or may comprise a nucleic acid sequence encoding an OATP of the invention or part thereof and one or more regulatory elements which differ from the regulatory elements that drive expression of the native protein. In another preferred embodiment, the animal has an OATP gene which is misexpressed or not expressed, e.g., a knockout. Such transgenic animals can serve as a model for studying disorders that are related to mutated or misexpressed OATPs of the present invention.

The invention still further provides a method for identifying a substance which is capable of binding a novel OATP of the invention, comprising reacting a novel OATP of the invention or part of the protein under conditions which permit the formation of a complex between the substance and a novel OATP protein or part of the protein, and assaying for substance-OATP complexes, for free substance, for non-complexed OATP, or for activation of an OATP.

An embodiment of the invention provides a method for identifying substrates which are capable of binding to a novel OATP protein of the invention, isoforms thereof, or part of the protein, said method comprising reacting a novel OATP protein of the invention, isoforms thereof, or part of the protein, with at least one substrate which potentially is capable of binding to the protein, isoform, or part of the protein, under conditions which permit the formation of substrate-transporter protein complexes, and assaying for substrate-transporter protein complexes, for free substrate, for non-complexed OATP protein, or for activation of an OATP. In a preferred embodiment of the method, substrates are identified which are capable of binding to and being transported by a novel OATP protein of the invention, isoforms thereof, or part of the protein.

Further described are methods for screening potentially useful pharmacological agonists or antagonists of the OATPs of the present invention. The method comprises testing potential agents by adding the agent to be tested to a cell expressing a novel OATP of the present invention in the presence of a compound known to be transported by an OATP of the invention, and measuring the augmentation or inhibition of transport of the known compound.

An OATP of the present invention is also useful to identify compounds that may be transported into an organ, e.g., the liver. Compounds that are found to be actively transported into the liver are useful as carriers for other therapeutics targeting the liver.

Further described is a composition which may include an OATP of the present invention, a fragment thereof (or a nucleic acid encoding said OATP or fragment thereof) and one or more additional components, e.g., a carrier, diluent or solvent. The additional component can be one that renders the composition useful for in vitro, in vivo, pharmaceutical or veterinary use.

Further described are agonists and antagonists of an OATP of the present invention. Pharmacological agonists or antagonists are useful to increase or decrease the flow of compounds transported by an OATP of the present invention. Said agonists and/or antagonists are preferably administered with an acceptable carrier, diluent or solvent.

Another aspect relates to a method of treating a mammal, e.g., a human, at risk for a disorder, e.g., a disorder characterized by aberrant or unwanted level or biological activity of an OATP of the present invention and to a method of treating a mammal, e.g., a human, at risk for disorders of the liver. Since OATP2 is expressed exclusively in the liver, compounds that are optimized for OATP2 are useful to target hepatic delivery. These compounds in themselves may be useful therapeutics, or may be useful to chaperone other therapeutic compounds to the liver. In addition, blocking OATP2-compound interactions could provide benefit by decreasing its first-pass extraction by the liver and, thus, increasing plasma concentrations and prolonging the systemic half-life of a drug.

Also within the scope of the present invention are fusion proteins comprising all or a portion of an OATP of the present invention.

The primary object of the present invention is the identification of a new human OATP, as identified by the nucleic acid and amino acid sequences disclosed herein. Additional objects of the invention are the methods of using the cDNA, the OATP protein, monoclonal antibodies specific for the novel OATPs, a fusion protein comprising a portion of the OATP protein of the present invention.

### Brief Description of the Figures

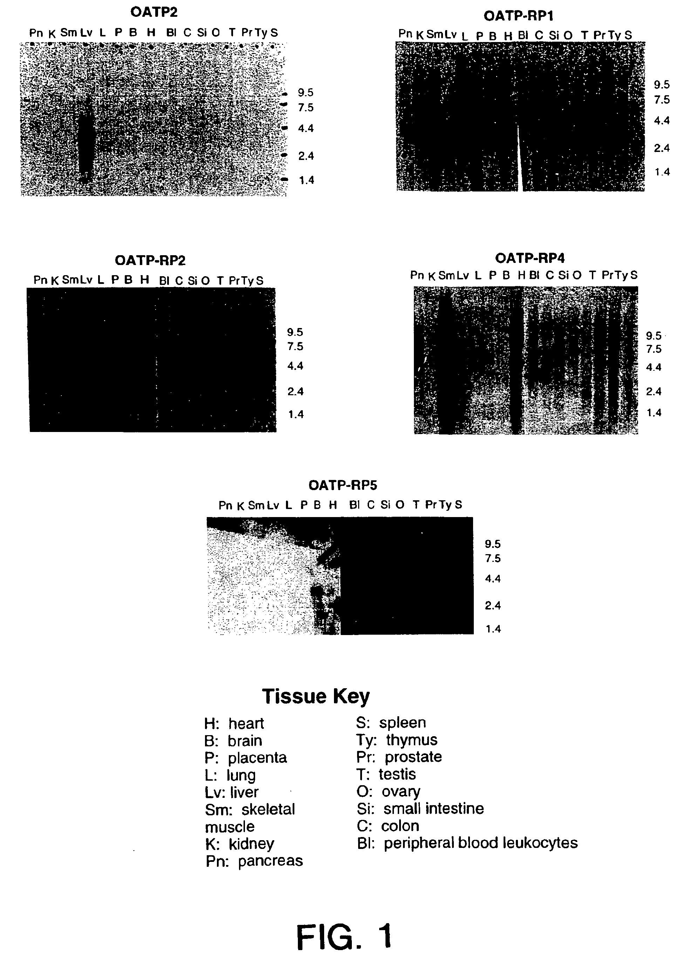

Figure 1 is a Northern blot showing the mRNA tissue distribution of OATP2, OATP-RP1, OATP-RP2, OATP-RP4, and OATP-RP5. The tissues corresponding to the abbreviations above the lanes are indicated below.

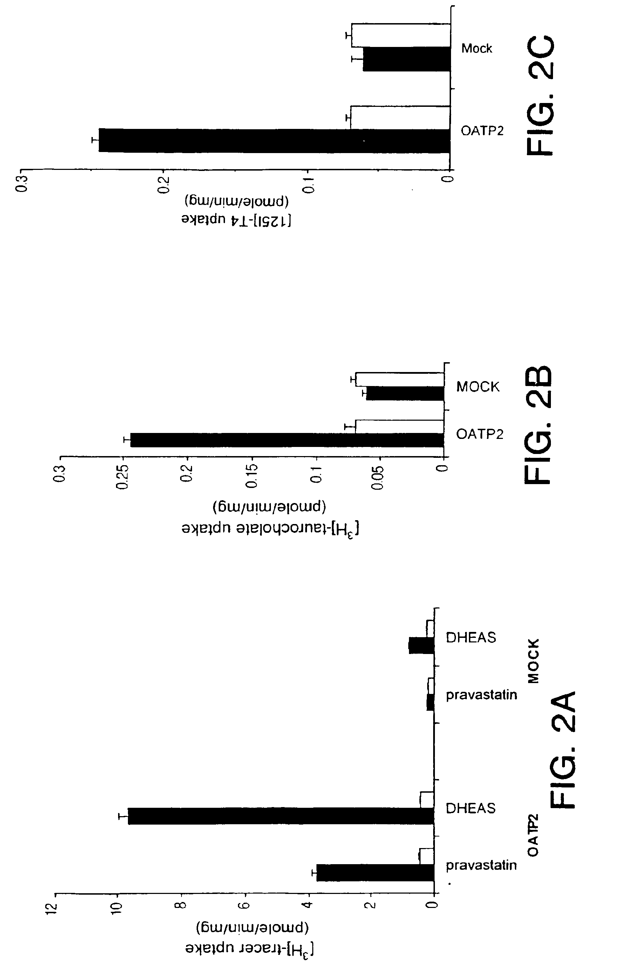

Figure 2 shows that OATP2 transports pravastatin, dehydroepiandosterone sulfate (DHEAS), taurocholate and thyroid hormone (T ). Figure 2A shows specific uptake of [³H]-pravastatin and [³H]-DHEAS. Figure 2B shows specific uptake of [³H]-taurocholate. Panel 2C shows specific uptake of [125I]-thyroid hormone (T4). The uptake of radiolabeled substrate for 5 minutes into cells transfected with pCEPOATP-RP1 or empty vector (MOCK) was determined in the absence (solid bars) and presence (open bars) of excess unlabeled substrate.

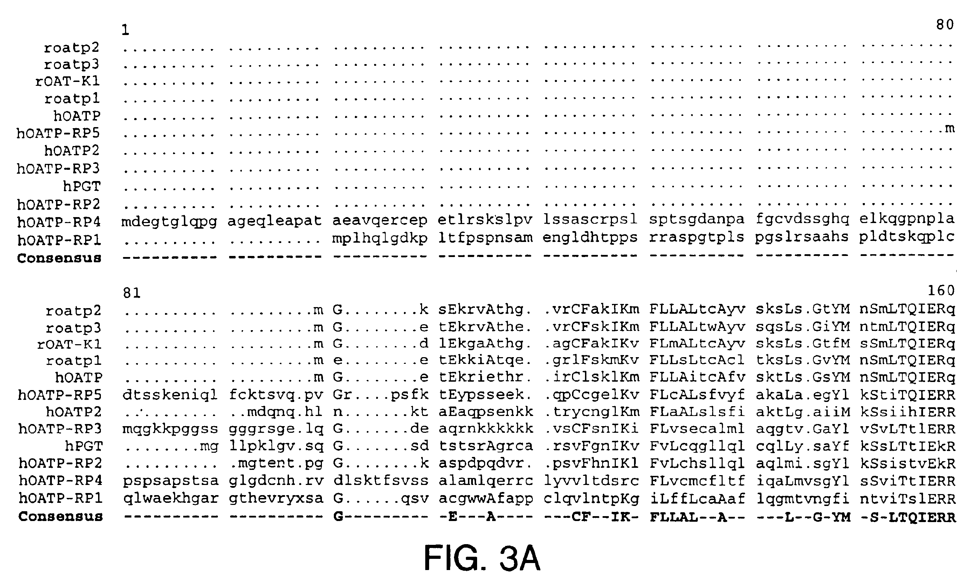

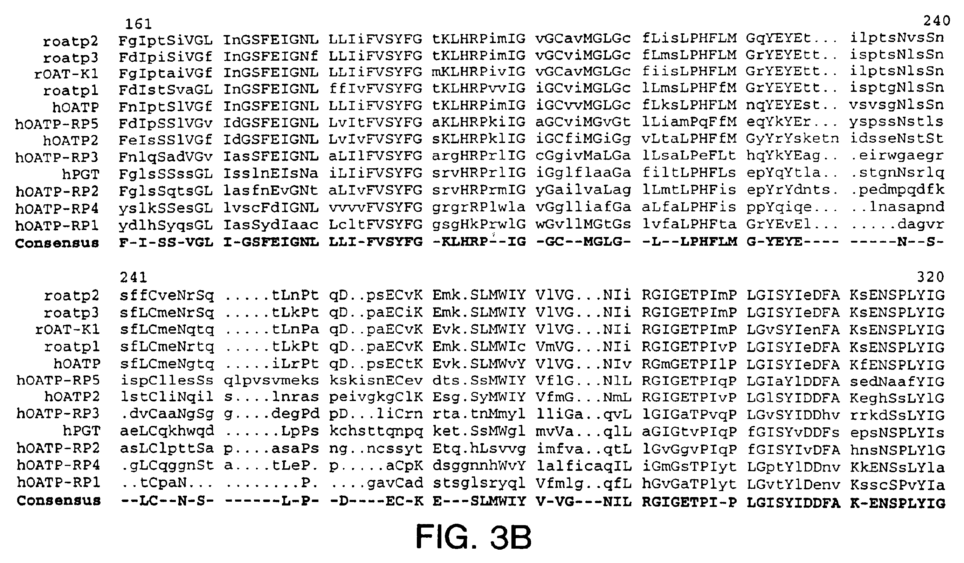

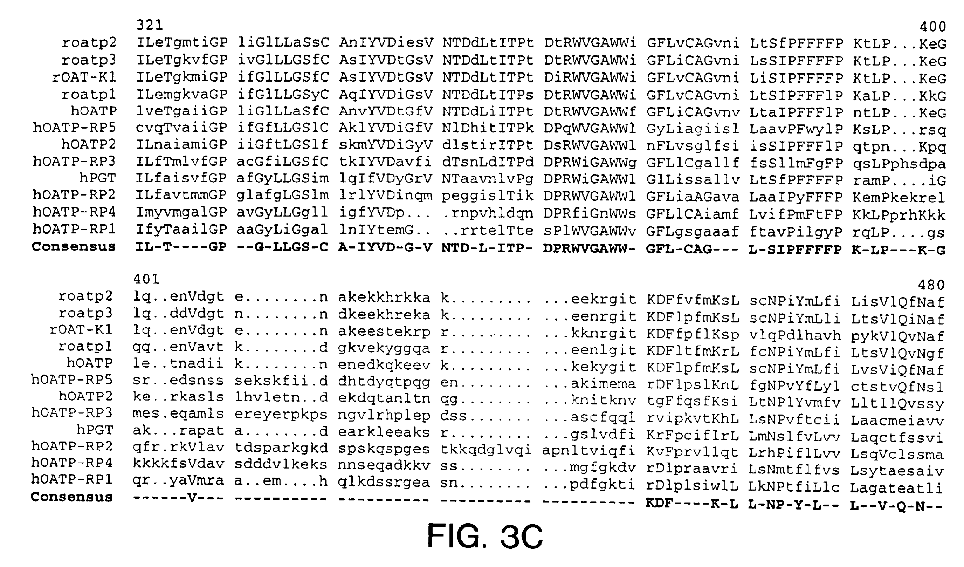

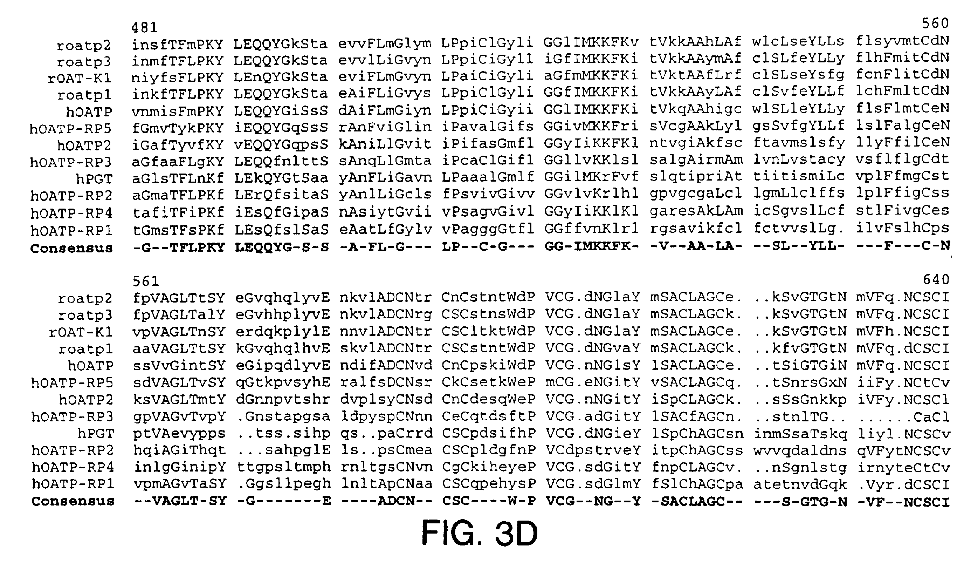

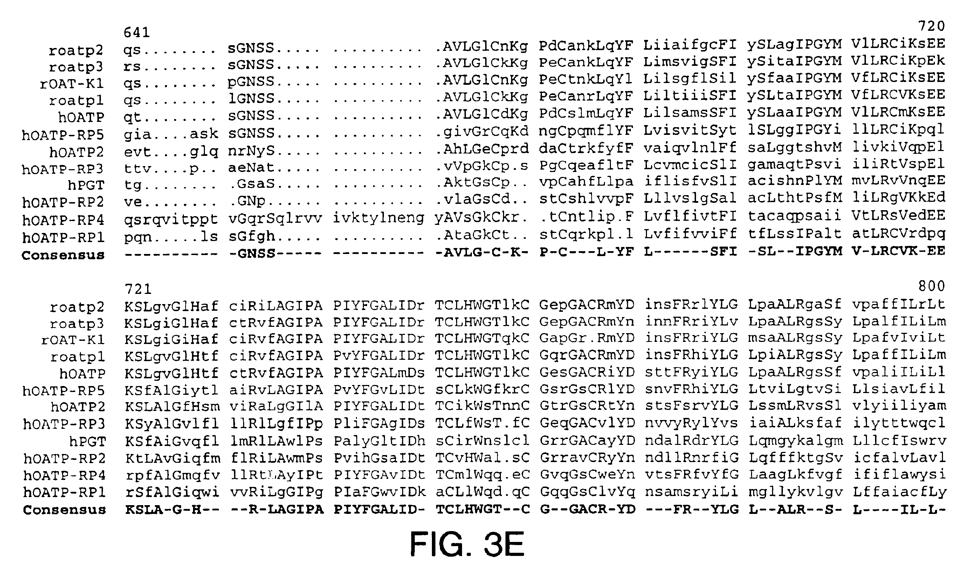

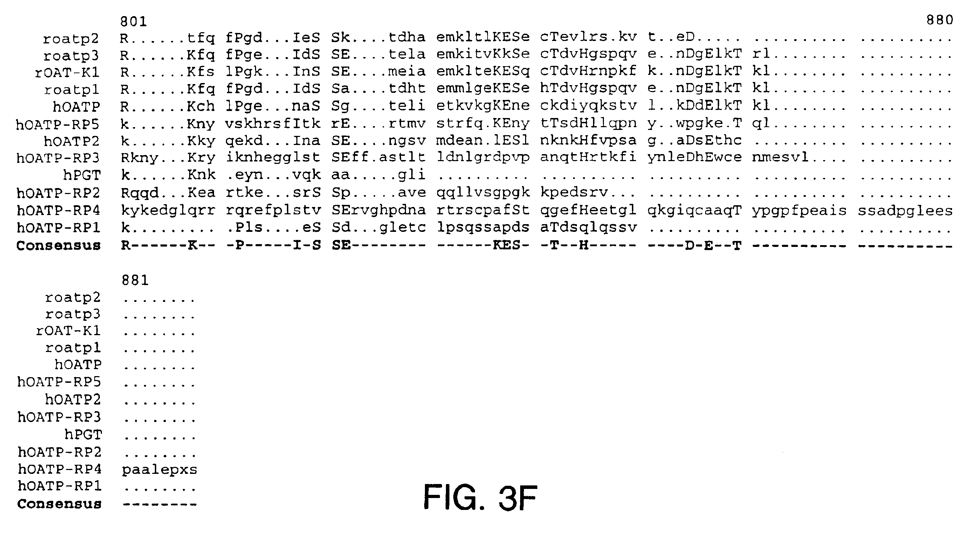

Figure 3 shows a sequence alignment of OATP family members. The protein sequences of human OATP2, OATP-RP1, OATP-RP2, OATP-RP3, OATP-RP4, and OATP-RP5 are aligned with the other known OATP family members. Also shown is a consensus sequence in bold. A consensus is indicated if at least 6 out of the 12 sequences are identical at a given position. A residue is capitalized if it agrees with the consensus.

### Detailed Description of the Invention

The following definitions apply to the terms used throughout this specification, unless otherwise defined in specific instances:
"cloning" - isolation of a particular gene from genetic material, for example a genome, genomic library, or cDNA library into a plasmid or other vector;
"coding region" - the region of a nucleic acid sequence that codes for an active protein;
"OATP" - organic anion transport protein;
"stringent conditions" (as used concerning nucleic acid hybridization)-Southern blotting washed in 0.1 X SSC and 0.1 % SDS at a temperature of at least about 65° C. See Maniatis et al., Molecular Cloning: A Laboratory Manual, Cold Spring Harbor Laboratory, Cold Spring Harbor, NY (1982); one skilled in the relevant art would recognize that less stringent conditions (e.g., 1X or 2X SSC, 0.1%SDS) may be employed in using the novel sequences disclosed herein to identify nucleic acid sequences encoding novel OATPs.
"Northern blotting"-a method of identifying particular RNA fragments by hybridization with a complementary nucleic acid, typically a cDNA or an oligonucleotide;
"open reading frame" or "ORF"-a DNA sequence containing a series of nucleotide triplets coding for amino acids and lacking any termination codes;
"plasmid"-cytoplasmic, autonomously replicating DNA elements found in microorganisms;
"promoter"-a region on DNA at which RNA polymerase binds and initiates transcription; and
"Southern blotting"-a method of identifying particular DNA fragments by hybridization with a complementary nucleic acid, typically a cDNA or an oligonucleotide;
"transport" - the movement of a substance across a biological membrane as determined by measuring the redistribution of such a substance across the membrane upon exposure to a transporter.

For definitions of other terms in this specification, see F. Sherman et al., Laboratory Course Manual for Methods in Yeast Genetics, Cold Spring Harbor Laboratory, Cold Spring Harbor, NY (1987) and Lewin, B., Genes IV, Oxford University Press, Oxford (1990). For the definitions of abbreviations, see Aldrichimica Acta, Vol. 17, No. 1 (1984).

### Use and utility

The amino acid sequences of the novel organic anion transport proteins of the present invention are aligned with known transporters of this family in Figure 3. The degree of sequence homology between the sequences of the present invention and known organic anion transporters indicates that the proteins of the present invention are organic anion transporters.

It is believed by those skilled in the art that OATP proteins may be involved in the transport of compounds into the liver. Persons of ordinary skill in the art can use the OATP proteins of the present invention to assay for agents that may increase or decrease the rate of transport of compounds into the liver, or for compounds that are transported by the OATPs of the present invention that are useful as carriers for other compounds that are desired to be carried to a specific organ (e.g., the liver).

Therefore, agents that increase or decrease the rate of substrate transport by the OATPs of the present invention, or agents identified as carriers, are useful in the treatment of liver disease.

Because some of the OATPs are organ specific/selective (e.g., OATP2 - liver; OATP-RP4 - heart and skeletal muscle, and OATP-RP5 - brain and testis), compound specificity is built into any specific substrate of these OATPs and into molecular carriers transported by these OATPs. An agent transported by the above OATPs of the present invention would thus be delivered to the tissues in which they are expressed and not to tissues lacking the above OATPs, thereby achieving tissue specific targeting.

The OATP nucleic acids of the present invention, or antisense nucleic acids, may be useful therapeutic or diagnostic agents. For such gene therapy, the nucleic acids may be incorporated into vectors and/or formulated as described below and in further detail in the art.

The present invention also provides a basis for diagnostic genetic screens for predicting response to drugs. At least one of the transporters disclosed and claimed herein is a transporter of a known drug (i.e., OATP2 transports pravastatin into hepatocytes). Other transporters disclosed herein may similarly transport additional drugs into tissues. Persons skilled in the art can: (1) screen the transporter genes for allelic variants (genotypes) in the general population by various sequencing methods; and (2) determine the association of these transporter genotypes in patients with response to the transported drug in clinical trials. Particular allelic variants may be more or less effective in transporting a drug, which would be related to drug efficacy. Thus, genotyping of the claimed transporters could form the basis of a clinical diagnostic test to predict a patient's response to drug therapy.

Persons skilled in the art can use the polypeptides and nucleic acids of this invention to prepare vectors, cells or cell lines, and antibodies. All of these are useful in assays for identification of OATP positive and negative modulators (i.e., agonists and/or antagonists) and OATP carriers. The term "positive modulator" as used herein refers to an agent or compound that increases the rate or amount of transport of a compound into an organ, e.g., the liver, or an agent or compound that decreases the rate or amount of transport of a compound into an organ. The term "negative modulator" refers to a compound that is joined to a second compound to prevent the second compounds transport into or out of cells. The term "carrier" as used herein refers to an agent or compound that is transported by an OATP of the present invention and that is capable of being joined to or associated with another compound to chaperone that other compound into an organ, e.g., the liver. A carrier includes an agent that is used to transport a compound into an organ that is otherwise not transported into said organ, and includes an agent that increases the transport of a compound into an organ that is capable of being transported by an OATP.

One can administer OATP modulators and carriers to various mammalian species, such as monkeys, dogs, cats, mice, rats, humans, etc. By known methods, persons skilled in the pharmaceutical art can incorporate OATP modulators and carriers in a conventional systemic dosage form, such as a tablet, capsule, elixir or injectable formulation. The above dosage forms will also include any necessary physiologically acceptable carrier material, excipient, lubricant, buffer, antibacterial, bulking agent (such as mannitol), anti-oxidants (ascorbic acid or sodium bisulfite) or the like.

### Process of preparation

### In general

This specification describes the cloning and functional expression of the full-length human cDNA clones of OATP2, i.e. the nucleic acid sequence of OATP2 (SEQ ID NO:1) and, the amino acid sequence of OATP2 (SEQ ID NO:2).

DNA clones comprising nucleotide sequences encoding the OATP described above were deposited with the American Type Culture Collection ("ATCC") (10801 University Blvd.. Manassas. VA 20110-2209) on April 20, 1999, and given the following ATCC Accession Numbers: 207209 (OATP-RP3), 207210 (OATP-RP4), 207211 (OATP-RP5). 207212 (OATP-RP2), 207213 (OATP2), and 207214 (OATP-RP1). The deposit(s) referred to herein will be maintained under the terms of the Budapest Treaty on the International Recognition of the Deposit of Microorganisms for purposes of Patent Procedure. These deposits are provided merely as convenience to those of skill in the art and are not an admission that a deposit is required under 35 U.S.C. §112. The sequence of the polynucleotides contained in the deposited materials, as well as the amino acid sequence of the of the polypeptides encoded thereby, are incorporated herein by reference and are controlling in the event of any conflict with any description of sequences herein. A license may be required to make, use or sell the deposited materials, and no such license is hereby granted.

### Nucleic acids

With the disclosed OATP gene sequences in hand, one skilled in the art can obtain OATP nucleic acids of this invention by known methods. Such methods include: (1) Southern and Northern blotting; (2) Western immunoblotting; (3) chemical synthesis; (4) synthesis by polymerase chain reaction (PCR) from primers; (5) expression cloning; and (6) subtractive cDNA cloning.

Preferred nucleic acid sequences of the present invention include the following (preferably the coding sequences as shown below):

OATP2 (SEQ ID NOS:1 and 2):

CGGACGCGTG GGCGGACGCG TGGGTCGCCC ACGCGTCCGA CTTGTTGCAG 50
TTGCTGTAGG ATTCTAAATC CAGGTGATTG TTTCAAACTG AGCATCAACA 100
ACAAAAACAT TTGTATGATA TCTATATTTC AATC ATG GAC CAA AAT CAA 149
M D Q N Q

CAT TTG AAT AAA ACA GCA GAG GCA CAA CCT TCA GAG AAT AAG 191
H L N K T A E A Q P S E N K

AAA ACA AGA TAC TGC AAT GGA TTG AAG ATG TTC TTG GCA GCT 233
K T R Y C N G L K M F L A A

CTG TCA CTC AGC TTT ATT GCT AAG ACA CTA GGT GCA ATT ATT 275
L S L S F I A K T L G A I I

ATG AAA AGT TCC ATC ATT CAT ATA GAA CGG AGA TTT GAG ATA 317
M K S S I I H I E R R F E I

TCC TCT TCT CTT GTT GGT TTT ATT GAC GGA AGC TTT GAA ATT 359
S S S L V G F I D G S F E I

GGA AAT TTG CTT GTG ATT GTA TTT GTG AGT TAC TTT GGA TCC 401
G N L L V I V F V S Y F G S

AAA CTA CAT AGA CCA AAG TTA ATT GGA ATC GGT TGT TTC ATT 443
K L H R P K L I G I G C F I

ATG GGA ATT GGA GGT GTT TTG ACT GCT TTG CCA CAT TTC TTC 485
M G I G G V L T A L P H F F

ATG GGA TAT TAC AGG TAT TCT AAA GAA ACT AAT ATC GAT TCA 527
M G Y Y R Y S K E T N I D S

TCA GAA AAT TCA ACA TCG ACC TTA TCC ACT TGT TTA ATT AAT 569
S E N S T S T L S T C L I N

CAA ATT TTA TCA CTC AAT AGA GCA TCA CCT GAG ATA GTG GGA 611
Q I L S L N R A S P E I V G

AAA GGT TGT TTA AAG GAA TCT GGG TCA TAC ATG TGG ATA TAT 653
K G C L K E S G S Y M W I Y

GTG TTC ATG GGT AAT ATG CTT CGT GGA ATA GGG GAG ACT CCC 695
V F M G N M L R G I G E T P

ATA GTA CCA TTG GGG CTT TCT TAC ATT GAT GAT TTC GCT AAA 737
I V P L G L S Y I D D F A K

GAA GGA CAT TCT TCT TTG TAT TTA GGT ATA TTG AAT GCA ATA 779
E G H S S L Y L G I L N A I

GCA ATG ATT GGT CCA ATC ATT GGC TTT ACC CTG GGA TCT CTG 821
A M I G P I I G F T L G S L

TTT TCT AAA ATG TAC GTG GAT ATT GGA TAT GTA GAT CTA AGC 863
F S K M Y V D I G Y V D L S

ACT ATC AGG ATA ACT CCT ACT GAT TCT CGA TGG GTT GGA GCT 905
T I R I T P T D S R W V G A

TGG TGG CTT AAT TTC CTT GTG TCT GGA CTA TTC TCC ATT ATT 947
W W L N F L V S G L F S I I

TCT TCC ATA CCA TTC TTT TTC TTG CCC CAA ACT CCA AAT AAA 989
S S I P F F F L P Q T P N K

CCA CAA AAA GAA AGA AAA GCT TCA CTG TCT TTG CAT GTG CTG 1031
P Q K E R K A S L S L H V L

GAA ACA AAT GAT GAA AAG GAT CAA ACA GCT AAT TTG ACC AAT 1073
E T N D E K D Q T A N L T N

CAA GGA AAA AAT ATT ACC AAA AAT GTG ACT GGT TTT TTC CAG 1115
Q G K N I T K N V T G F F Q

TCT TTT AAA AGC ATC CTT ACT AAT CCC CTG TAT GTT ATG TTT 1157
S F K S I L T N P L Y V M F

GTG CTT TTG ACG TTG TTA CAA GTA AGC AGC TAT ATT GGT GCT 1199
V L L T L L Q V S S Y I G A

TTT ACT TAT GTC TTC AAA TAC GTA GAG CAA CAG TAT GGT CAG 1241
F T Y V F K Y V E Q Q Y G Q

CCT TCA TCT AAG GCT AAC ATC TTA TTG GGA GTC ATA ACC ATA 1283
P S S K A N I L L G V I T I

CCT ATT TTT GCA AGT GGA ATG TTT TTA GGA GGA TAT ATC ATT 1325
P I F A S G M F L G G Y I I

AAA AAA TTC AAA CTG AAC ACC GTT GGA ATT GCC AAA TTC TCA 1367
K K F K L N T V G I A K F S

TGT TTT ACT GCT GTG ATG TCA TTG TCC TTT TAC CTA TTA TAT 1409
C F T A V M S L S F Y L L Y

TTT TTC ATA CTC TGT GAA AAC AAA TCA GTT GCC GGA CTA ACC 1451
F F I L C E N K S V A G L T

ATG ACC TAT GAT GGA AAT AAT CCA GTG ACA TCT CAT AGA GAT 1493
M T Y D G N N P V T S H R D

GTA CCA CTT TCT TAT TGC AAC TCA GAC TGC AAT TGT GAT GAA 1535
V P L S Y C N S D C N C D E

AGT CAA TGG GAA CCA GTC TGT GGA AAC AAT GGA ATA ACT TAC 1577
S Q W E P V C G N N G I T Y

ATC TCA CCC TGT CTA GCA GGT TGC AAA TCT TCA AGT GGC AAT 1619
I S P C L A G C K S S S G N

AAA AAG CCT ATA GTG TTT TAC AAC TGC AGT TGT TTG GAA GTA 1661
K K P I V F Y N C S C L E V

ACT GGT CTC CAG AAC AGA AAT TAC TCA GCC CAT TTG GGT GAA 1703
T G L Q N R N Y S A H L G E

TGC CCA AGA GAT GAT GCT TGT ACA AGG AAA TTT TAC TTT TTT 1745
C P R D D A C T R K F Y F F

GTT GCA ATA CAA GTC TTG AAT TTA TTT TTC TCT GCA CTT GGA 1787
V A I Q V L N L F F S A L G

GGC ACC TCA CAT GTC ATG CTG ATT GTT AAA ATT GTT CAA CCT 1829
G T S H V M L I V K I V Q P

GAA TTG AAA TCA CTT GCA CTG GGT TTC CAC TCA ATG GTT ATA 1871

E L K S L A L G F H S M V I

CGA GCA CTA GGA GGA ATT CTA GCT CCA ATA TAT TTT GGG GCT 1913
R A L G G I L A P I Y F G A

CTG ATT GAT ACA ACG TGT ATA AAG TGG TCC ACC AAC AAC TGT 1955
L I D T T C I K W S T N N C

GGC ACA CGT GGG TCA TGT AGG ACA TAT AAT TCC ACA TCA TTT 1997
G T R G S C R T Y N S T S F

TCA AGG GTC TAC TTG GGC TTG TCT TCA ATG TTA AGA GTC TCA 2039
S R V Y L G L S S M L R V S

TCA CTT GTT TTA TAT ATT ATA TTA ATT TAT GCC ATG AAG AAA 2081
S L V L Y I I L I Y A M K K

AAA TAT CAA GAG AAA GAT ATC AAT GCA TCA GAA AAT GGA AGT 2123
K Y Q E K D I N A S E N G S

GTC ATG GAT GAA GCA AAC TTA GAA TCC TTA AAT AAA AAT AAA 2165
V M D E A N L E S L N K N K

CAT TTT GTC CCT TCT GCT GGG GCA GAT AGT GAA ACA CAT TGT 2207
H F V P S A G A D S E T H C

TAA GGGGAGAAAA AAAGCCACTT CTGCTTCTGT GTTTCCAAAC AGCATTGCAT 2260
*

TGATTCAGTA AGATGTTATT TTTGAGGAGT TCCTGGTCCT TTCACTAAGA 2310
ATTTCCACAT CTTTTATGGT GGAAGTATAA ATAAGCCTAT GAACTTATAA 2360
TAAAACAAAC TGTAGGTAGA AAAAATGAGA GTACTCATTG TTACATTATA 2410
GCTACATATT TGTGGTTAAG GTTAGACTAT ATGATCCATA CAAATTAAAG 2460
TGAGAGACAT GGTTACTGTG TAATAAAAGA AAAAATACTT GTTCAGGTAA 2510
TTCTAATTCT TAATAAAACA AATGAGTATC ATACAGGTAG AGGTTAAAAA 2560
GGAGGAGCTA GATTCATATC CTAAGTAAAG AGAAATGCCT AGTGTCTATT 2610
TTATTAAACA AACAAACACA GAGTTTGAAC TATAATACTA AGGCCTGAAG 2660
TCTAGCTTGG ATATATGCTA CAATAATATC TGTTACTCAC ATAAAATTAT 2710
ATATTTCACA GACTTTATCA ATGTATAATT AACAATTATC TTGTTTAAGT 2760
AAATTTAGAA TACATTTAAG TATTGTGGAA GAAATAAAGA CATTCCAATA 2810
TTTGCAAAAA AAAAAAAAAA 2830

GCATAAATAT ATATTTATGG ACACACAGTT TGCATCAGAA CGTGTTTATA 2554
GAATGTGTTT TATACCCGAT CGTGTGTGGT GTGCGTGAGG ACAAACTCCG 2604
CAGGGGCTGT GAATCCCACT GGGAGGGCGG CGGGCCTGCA GCCCGAGGAA 2654
GGCTTGTGTG TCCTCAGTTA AAACTGTGCA TATCGAAATA TATTTTGTTA 2704
TTTAAGCCTG CGAAAAAAAA AAAAAAAAAA AAAAAAAAAA AAAAAAAAAA 2754
AAAAAAAAA 2763

Persons skilled in the art can also modify the nucleic acid coding for the OATP of the present invention to prepare useful mutations. For example, one may modify the sequence to provide additional restriction endonuclease recognition sites in the nucleic acid. Such mutations may be silent or may change the amino acid encoded by the mutated codon. One can prepare these modified nucleic acids, for example, by mutating the nucleic acid coding for an OATP of the present invention to result in deletion, substitution, insertion, inversion or addition of one or more amino acids in the encoded polypeptide. For methods of site-directed mutagenesis, see Taylor, J. W. et al. (1985), Nucl. Acids Res. 13, 8749-64 and Kunkel, J. A. (1985), Proc. Natl. Acad. Sci. USA 82: 482-92. In addition, kits for site-directed mutagenesis are available from commercial vendors (e.g., BioRad Laboratories, Richmond, CA; Amersham Corp., Arlington Heights, IL). For disruption, deletion and truncation methods, see Sayers, J. R. et al. (1988), Nucl. Acids Res. 16: 791-800.

Also described are modified nucleic acids, including (1) alternative splice exon variants; (2) allelic variants; and (3) chimeric proteins in which the fusion construct comprises an OATP or fragment thereof. Such modified nucleic acids can be obtained by persons of ordinary skill in the art when armed with the present disclosure.

### Expression vectors

This invention further concerns expression vectors comprising a nucleotide sequence encoding an OATP of the present invention. Preferably, the expression vectors comprise all or a portion of the nucleic acid sequence as shown in SEQ ID NO:1; preferred is a nucleotide sequence encoding an OATP as shown above (i.e., the coding region).

Expression vectors are usually plasmids, but the invention includes other vector forms that serve equivalent functions and become known in the art subsequently hereto. A person skilled in the art might also stably integrate a sequence encoding an OATP into the chromosome of an appropriate host cell.

Expression vectors typically contain regulatory elements capable of affecting expression of an OATP. These regulatory elements can be heterologous or native OATP elements. Typically, a vector contains an origin of replication, a promoter, and a transcription termination sequence. The vector may also include other regulatory sequences, including mRNA stability sequences, which provide for stability of the expression product; secretory leader sequences, which provide for secretion of the expression product; environmental feedback sequences, which allow expression of the structural gene to be modulated (e.g., by the presence or absence of nutrients or other inducers in the growth medium); marking sequences, which are capable of providing phenotypic selection in transformed host cells; restriction sites, which provide sites for cleavage by restriction endonucleases; and sequences which allow expression in various types of hosts, including prokaryotes, yeasts, fungi, plants and higher eukaryotes.

An expression vector of this invention is at least capable of directing the replication, and preferably the expression, of the nucleic acids and protein of this invention. Suitable origins of replication include, for example, the Col E1, the SV4O viral, Epstein Barr viral, and the M13 origins of replication. Suitable promoters include, for example, the cytomegalovirus promoter, the lacZ promoter, the gal10 promoter and the Autographa californica multiple nuclear polyhedrosis virus (AcMNPV) polyhedral promoter. Suitable termination sequences include, for example, the bovine growth hormone, SV40, lacZ and AcMNPV polyhedral polyadenylation signals. Examples of selectable markers include neomycin, ampicillin, and hygromycin resistance and the like.

Persons skilled in the art may insert DNA encoding an OATP of the present invention into several commercially available vectors. Examples include vectors compatible with mammalian cells, such as pcDNA3 or pCEP4; baculovirus vectors such as pBlueBac; prokaryotic vectors such as pcDNA2; and yeast vectors such as pYes2. For vector modification techniques, see Sambrook et al. (1989), Molecular Cloning: A Laboratory Manual, Second Edition, Cold Spring Harbor Laboratory, Cold Spring Harbor, NY.

### Host cells

This invention additionally concerns host cells containing an expression vector that comprises a sequence encoding OATP2, of the present invention. The host cells preferably contain an expression vector which comprises all or part of the DNA sequence having the nucleotide sequence substantially as shown in SEQ ID NO:1 particularly the coding regions thereof. Suitable host cells include both prokaryotic cells (e.g., E. coli strains HB101, DH5a, XL1 Blue, Y1090 and JM101) and eukaryotic cells (e.g., Spodoptera frugiperda insect cells. CHO cells, COS-7 cells, HEK 293 cells, human skin fibroblasts, and S. cerevisiae cells).

Persons skilled in the art may introduce expression vectors into host cells by various methods known in the art. Exemplary methods are transfection by calcium phosphate precipitation, electroporation, liposomal fusion, nuclear injection, and viral or phage infection. One may then culture the host cell under conditions permitting expression of large amounts of OATP.

One may identify such modified host cells by any of five general approaches:
(a) DNA-DNA hybridization with probes complementary to the sequence encoding an OATP (Southern blotting).
(b) detection of marker gene functions, such as thymidine kinase activity, resistance to antibiotics, and the like. A marker gene can be placed in the same plasmid as an OATP sequence under the regulation of the same or a different promoter.
(c) detection of mRNA transcripts by hybridization assays (e.g., Northern blotting or a nuclease protection assay using a probe complementary to the RNA sequence).
(d) immunodetection of gene expression (e.g., by Western blotting with antibody to OATP).
(e) PCR with primers homologous to expression vector sequences or sequences encoding OATP. The PCR produces a DNA fragment of predicted length, indicating incorporation of the expression system in the host cell.

Persons skilled in the art may determine DNA sequences by various known methods. See, for example, the dideoxy chain termination method in Sanger et al. (1977), Proc. Natl. Acad. Sci. USA 74: 5463-7 and the Maxam-Gilbert method in Maxam-Gilbert (1977), Proc. Natl. Acad. Sci. USA 74: 560-4.

One may use the host cells of this invention in a variety of ways that are now apparent. One may use the cells to screen for compounds that bind to or otherwise modulate or regulate the function of an OATP of the present invention, which would be useful for modulation, for example activation or inactivation, of OATP2, OATP-RP2, OATP-RP3, OATP-RP4, OATP-RP5 or OATP-RP1 activity; to study signal transduction mechanisms and protein-protein interactions; and to prepare OATP for the uses described below.

Not all expression vectors and DNA regulatory sequences will function equally well to express the DNA sequences of this invention. Neither will all host cells function equally well with the same expression system. However, one of ordinary skill in the art may make a selection among expression vectors, DNA regulatory sequences, and host cells using the guidance provided herein without undue experimentation and without departing from the scope of the invention.

### Polypeptides

This invention further concerns polypeptides comprising all or a portion of the amino acid sequences of the OATP of the present invention. The inventors prefer polypeptides comprising all or a portion of the amino acid sequences shown as in SEQ ID NO:2 (OATP2).

Where a portion of an OATP of the present invention is used, preferably the portion exhibits the same biological activity of the OATP from which the portion is derived. For example, and within the scope of the invention, are polypeptides that comprise all or a portion of OATP2 that exhibit transport activity. The portions may contain one or more mutations so that the protein(s) fail(s) to exhibit transport activity, but that can be used to screen for compounds that will modulate or bind to the protein or portion thereof.

Persons having ordinary skill in the art may prepare these polypeptides by methods known in the art. For example, one may use chemical synthesis, such as the solid phase procedure described by Houghton et al. (1985), Proc. Natl. Acad. Sci. 82: 5131-5. Another method is in vitro translation of mRNA. One may also produce the polypeptides in the above-described host cells, which is the preferred method. For example, one may synthesize DNA comprising all or a portion of SEQ ID NO:1 by PCR as described above, insert the synthesized DNA into an expression vector, transform a host cell with the expression vector, and culture the host cell to produce the desired polypeptides.

Persons skilled in the art can isolate and purify such polypeptides by any one of several known techniques: for example, ion exchange chromatography, gel filtration chromatography and affinity chromatography. Such techniques may require modification of the protein. For example, one may add a histidine tag to the protein to enable purification on a nickel column.

Persons skilled in the art can use the polypeptides of the invention in a wide variety of ways. For example, one may use them to generate polyclonal or monoclonal antibodies. One may then use such antibodies for immunodetection (e.g., radioimmunoassay, enzyme immunoassay, or immunocytochemistry), immunopurification (e.g., affinity chromatography) of polypeptides from various sources, or immunotherapy.

Persons skilled in the art may make modified OATP polypeptides by known techniques. Such modifications may cause higher or lower activity, permit higher levels of protein production, or simplify purification of the protein. Such modifications may help identify specific OATP amino acids involved in binding, which in turn may help rational drug design of OATP modulators. One can make amino acid substitutions based on similarity in polarity, charge, solubility, hydrophobicity, hydrophilicity and/or the amphipathic nature of the residues involved. For example, negatively charged amino acids include aspartic acid and glutamic acid; positively charged amino acids include lysine and arginine; amino acids with uncharged polar head groups or nonpolar head groups having similar hydrophilicity values include the following: leucine, isoleucine, valine, glycine, alanine; asparagine, glutamine; serine, threonine; phenylalanine, tyrosine.

Analogs may include proteins that differ from the novel OATPs of the present invention (or biologically active fragments thereof) by one or more conservative amino acid substitutions or by one or more non-conservative amino acid substitutions, deletions or insertions which do not abolish the biological activity of the analog. Conservative substitutions typically include the substitution of one amino acid for another with similar characteristics, e.g., substitutions within the following groups: valine, glycine; glycine, alanine; valine, isoleucine, leucine; aspartic acid, glutamic acid; asparagine, glutamine; serine, threonine; lysine, arginine; and phenylalanine, tyrosine. Other conservative amino acid substitutions can be taken from the table below.

**Table 1**

| Conservative amino acid replacements | | |
|---|---|---|
| **For Amino Acid** | **Code** | **Replace with any of:** |
| Alanine | A | D-Ala, Gly, beta-Ala, L-Cys, D-Cys |
| Arginine | R | D-Arg, Lys, D-Lys, homo-Arg, D-homo-Arg, Met, Ile, D-Met, D-Ile, Om, D-Orn |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, β-Ala, Acp |
| Isoleucine | I | D-Ile, Val, D-Val, Leu, D-Leu, Met, D-Met |
| Leucine | L | D-Leu, Val, D-Val, Met. D-Met |
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-1-thioazolidine-4-carboxylic acid, D- or L-1-oxazolidine-4-carboxylic acid |
| Serine | S | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa. His, D-His |
| Valine | V | D-Val, Leu, D-Leu, Ile, D-Ile, Met, D-Met |

Other analogs are those with modifications which increase protein or peptide stability; such analogs may contain, for example, one or more non-peptide bonds (which replace the peptide bonds) in the protein or peptide sequence. Further analogs include residues other than naturally occurring L-amino acids. e.g., D-amino acids or non-naturally occurring or synthetic amino acids, e.g., B or γ amino acids.

The inventors contemplate a number of other variations of the above-described polypeptides. Such variations include salts and esters of the polypeptides, as well as precursors of the aforementioned polypeptides (e.g., having N-terminal substituents such as methionine, N-formylmethionine and leader sequences).

### Method for detecting nucleic acids

The present invention further concerns a method for detecting nucleic acids encoding the OATP protein. In this method, a person of ordinary skill in the art (a) contacts nucleic acids of unknown sequence with a nucleic acid having a sequence complementary to a known coding sequence (e.g., a sequence of at least about 10 nucleotides from, e.g., SEQ ID NO:1, particularly the coding regions thereof), wherein the latter nucleic acid has a detectable marker; and (b) determines the presence of marker bound to any of the nucleic acids of unknown sequence. The presence of bound marker indicates the presence of the desired nucleic acids. One can apply this method to detect OATP nucleic acids from other tissues (which may have different regulatory elements) and nucleic acids from other species (e.g., monkey).

Persons of ordinary skill in the art generally know how to obtain nucleic acids to be analyzed in this method. For genomic DNA, one can rapidly freeze tissue, crush the tissue into readily digestible pieces, and incubate the crushed tissue in proteinase K and SDS to degrade most cellular proteins. One can then deproteinize the genomic DNA by successive phenol/chloroformiisoamyl alcohol extractions, recover DNA by ethanol precipitation, dry it and resuspend it in buffer. For RNA, one can lyse cultured cells in 4M guanidinium solution, draw the lysate through a 20-gauge needle, pellet the RNA through a cesium chloride step gradient, and remove the supernatant. The pellet should contain purified RNA.

The detectable marker may be a radioactive ion linked to one of the nucleotides of the complementary nucleic acid. Common radioactive labels are ³²P and ³⁵S, although one may also use other labels such as biotin. Persons skilled in the art are aware of various methods to attach the labels to the complementary nucleic acid (e.g., the random primer method for attachment of ³²P or ³⁵S).

Persons of ordinary skill in the art generally know how to carry out such a method of detecting nucleic acids. For example, one may perform a Southern or northern blot using a radiolabeled OATP complementary oligonucleotide probe. One can then detect hybridization by autoradiography. Depending on the marker, one may also use other detection methods (e.g., spectrophotometry).

### Methods for detecting OATP modulators and compounds transported by the OATP of the present invention

This invention further concerns methods for detecting modulators of the OATP of the present invention, as well as methods for detecting compounds that are transported by the OATP of the present invention (e.g., compounds that are transported into the liver that may be used as carriers for other compounds). A screen for OATP modulators entails detecting binding of molecules (e.g., polypeptides, natural products, synthetic compounds) in cells expressing OATP protein. Alternatively, a screen for OATP positive modulators and/or negative modulators entails detecting the augmentation and/or inhibition of transport of a known compound. A screen for OATP-transported compounds entails detecting the transport of molecules (e.g., polypeptides, natural products, synthetic compounds) by an OATP.

Cloning and sequencing of the OATP of the present invention enables construction of cells useful in screening for natural products and synthetic compounds that bind to, modulate, and/or are transported by OATP activity. A process for detecting OATP modulators requires transforming a suitable vector into compatible host cells as described previously herein. One treats such transformed cells with test substances (e.g., synthetic compounds or natural products), and then measures activity in the presence and absence of the test substance.

### OATP Assay

An assay for the measurement of OATP activity is performed as follows:
HEK293 cells are plated in Dulbeccos Modified Eagles Medium (DMEM) plus 10% fetal bovine serum plus penecillin and streptomycin, in poly-d-lysine coated dishes and co-transfected with OATP transporter expression plasmids using Lipofectamine Plus (Life Technologies. Inc.). The cells and media are assayed for substrate transport 24 hours later. Alternatively, cell lines engineered to stably express OATPs could be plated and assayed directly without transfection. To measure transport, media is removed and monolayers are assayed in triplicate by washing once in serum-free DMEM and adding the same medium containing [³H]-substrate alone or in the presence of various concentrations of unlabeled test compounds. For OATP2, the [³H]-substrate could be [³H]-pravastatin, [³H]-taurocholate, or [³H]-dehydroepiandrosterone sulfate, or [¹²⁵I]-thyroid hormone (T4). Monolayers are incubated at room temperature for 5 to 10 minutes depending on the transporter.
   Then the cells are rapidly washed once with ice cold DMEM containing 5% BSA, twice with DMEM plus 0.1 % BSA and once with DMEM alone. Cells are lysed in 0.1 N NaOH and a fraction of the lysate is used to determine radiolabel incorporation by liquid scintillation counting, and another is used to determine protein concentration in the lysate using the Bradford assay with BSA as a standard. The transport activity is expressed as moles of substrate transported into cells/mg of cell protein/minute.

### Drug Targeting

Also included within the present invention is tissue expression of an OATP of the present invention. The OATPs of the present invention are also useful for targeting drugs to certain organs that express an OATP described herein (e.g., the liver), and for modulating the concentration of endogenous substrates.

For example, the novel organic anion transporter disclosed herein, OATP2, represents a potential therapeutic target due to its ability to modulate the cellular uptake and potential secretion of a several biologically important organic anions, including bile acids and the androgen hormone dehydroepiandrosterone sulfate ("DHEAS"). Furthermore, since OATP2 transports at least one drug (i.e. pravastatin), and other members of this family are known to transport a variety of other xenobiotics, this transporter could be exploited to optimize the delivery of drugs into liver and away from other tissues.

OATP2 is unique among the OATP family, in that it is the only known organic anion transporter that is expressed exclusively in the liver. Thus, drugs optimized for this transporter could be targeted for hepatic delivery with greater selectivity than with any other known transporter. To generalize this approach, it may be possible to identify a small molecule "adaptor" that is efficiently recognized and transported by OATP2 (an OATP2-transported compound) that could be appended to other drugs for hepatic targeting even if the parent compound is not transported by OATP2.

Alternatively, if a therapeutic compound is taken up into the liver entirely or substantially by OATP2, one could inhibit hepatic clearance and thereby elevate circulating concentrations, or increase the compounds half-life in the periphery, by adding a functionality to said compound that disallows transport by OATP2. Likewise, if an endogenous substance utilizes OATP2 for liver uptake and clearance from the circulation, a competitive or non-competitive OATP2 inhibitor could elevate plasma levels of said substance. As an example, DHEAS is an adrenal androgen that declines with age and on the basis of some animal data, it has been suggested that replacement of DHEAS deficiency may stimulate age-related immune deficiencies, increase cognitive function and insulin sensitivity, and maintain bone mass. Inhibiting the hepatic clearance of endogenous DHEAS through blocking its interactions with OATP2 could result in elevated hormone levels in the absence of hormone supplementation.

With the information provided herein, one skilled in the art is able to identify molecules, both naturally occurring and synthetic (including therapeutic drugs), that are transported by the OATP2 disclosed herein. OATPs as a class generally exhibit broad substrate specificity ("polyspecific" transporters). Thus, it is anticipated that many additional substrates of these transporters will be identified.

### Gene Therapy

Persons skilled in the art can also use sense and antisense nucleic acid molecules as therapeutic agents for OATP-related indications. One may construct vectors that direct the synthesis of the desired DNA or RNA or formulate the nucleic acid as described in the art.

Several references describe the usefulness of antisense molecule. See Toulme and Helene (1988), Gene 72: 51-8; Inouye (1988), Gene, 72: 25-34; Uhlmann and Peyman (1990), Chemical Reviews 90: 543-584; Biotechnology Newswatch (January 15, 1996), p. 4: Robertson. Nature Biotechnology 15: 209 (1997); Gibbons and Dzau (1996), Science 272: 689-93. One can design them based on genomic DNA and/or cDNA, 5' and 3' flanking control regions, other flanking sequences, intron sequences, and nonclassic Watson and Crick base pairing sequences used in formation of triplex DNA. Such antisense molecules include antisense oligodeoxyribonucleotides, oligoribonucleotides, oligonucleotide analogues, and the like, and may comprise at least about 15 to 25 bases.

Antisense molecules may bind noncovalently or covalently to the OATP DNA or RNA. Such binding could, for example, cleave or facilitate cleavage of OATP DNA or RNA, increase degradation of nuclear or cytoplasmic mRNA, or inhibit transcription, translation, binding of transactivating factors, or pre-mRNA splicing or processing. Antisense molecules may also contain additional functionalities that increase stability, transport into and out of cells, binding affinity, cleavage of the target molecule, and the like. All of these effects would decrease expression of OATP protein and thus make the antisense molecules useful as OATP modulators.

Full length clones for each of the above genes were obtained using the Gene Trapper cDNA Positive Selection System (LifeTechnologies, Inc.). In this procedure, a single or multiple oligonucleotides complementary to each of the EST contigs or individual EST sequences, were biotinylated at the 3'-end and used to hybridize to a single-stranded human cDNA library constructed in pCMVSport2 (LifeTechnologies, Inc.). The sequence of oligonucleotides used for each gene as well as the tissue source of the libraries screened are shown in Table 2.

**Table 2**

| Oligonucleotides used to screen for OATP Full length cDNAs using Gene-Trapper Selection | | | |
|---|---|---|---|
| **Gene** | **Biotinylated capture oligonucleotide(s) used** | **Seq ID number of oligonucleotide** | **Human cDNA library screened** |
| OATP2 | 5'-ACCCTGTCTAGCAGGTTGCA-3' | 13 | liver |
| OATP-RP1 | 5'-CTGTCGGAGTCTTCAGATG-3' | 14 | brain |
| OATP-RP2 | 5'-TCCATCACAGCCTCCTACGC-3' | 15 | liver |
| OATP-RP3 | 5'-TGCCTCTACTCTGACCCTAG-3' | 16 | heart |
| OATP-RP4 | 5'-GGAGCAGTCATTGACACCAC-3' | 17 | heart |
| | 5'-TGCTGGGAGTACAACGTGACG-3' | 18 | |
| | 5'-ACAAGGAGGATGGACrGCAG-3' | 19 | |
| OATP-RP5 | 5'-CAGGAATCCCAGCTCCAGTG-3' | 20 | brain |
| | 5'-GCTACAACCCAACTACTGGC-3' | 21 | |
| | 5'-GGGAcrAACTGTGATACTGG-3' | 22 | |

Hybrids between the biotinylated oligonucleotides and single-stranded cDNA were captured on streptavidin-coated paramagnetic beads. After washing, the captured single-stranded cDNA targets was released from the biotinylated oligonucleotides and converted to dsDNA by DNA polymerase using the corresponding unbiotinylated oligonucleotide. Following transformation and plating, several positive clones for each gene were identified by PCR analysis. Full-length cDNA clones were identified by sequencing. In the case of OATP-RP1, a partial cDNA was obtained by the above technique (pSP-RP1A). Another cDNA clone that was part of the OATP-RP1 contig was identified by searching the public EST databases (Genbank accession number AI027850). An EcoRI-NotI fragment of this clone containing the first 477 nucleotides of OATP-RP1 (SEQ ID NO: 11) (obtained from Research Genetics, Inc.) was ligated to EcoRI-Not I digested pSP-RP1A to generate the full length sequence.

Two polymorphic positions were identified when sequencing multiple OATP-RP4 cDNA clones. Thus, nucleotide number 713 of SEQ ID NO: 7 can be either a C, encoding Leu in SEQ ID NO:8, or a T, encoding a Phe in SEQ ID NO:8. Similarly, nucleotide number 2397 of SEQ ID NO: 7 can be either a G, encoding a Gly in SEQ ID NO:8 , or a T, encoding a Val in SEQ ID NO:8.

For expression studies, OATP2 cDNA was cloned into the expression vector pCEP4βR, a modified form of pCEP4 (Invitrogen, Inc.) in which the CMV promoter-driven expression cassette has been inverted, and used in transient transfections. To accomplish this, OATP2 cDNA in pCMVSport2, correponding to nucleotides 59 through 2361 of SEQ ID NO:1, was excised by digestion with KpnI and NotI. This fragment was cloned into KpnI-NotI digested pCEP4βR. This clone, pCEP-OATP2 was used for transient transfection expression studies.

### Example 2

### Tissue and cellular distribution of OATP2, OATP-RP1, OATP-RP2, OATP-RP4, and OATP-RP5

The tissue distribution of OATP2, OATP-RP1, OATP-RP2, OATP-RP4, and OATP-RP5 expression was determined by Northern blotting of poly A+ RNA from a variety of human tissues (Figure 1). Transporters of this family previously described in the literature, namely human OATP, rat oatp1, rat oatp2 and rat oatp3, are all expressed in liver, kidney and brain. All of the above transport bile acids as well as a variety of other substrates that are specific for subsets of these transporters. In contrast, the expression of OATP2, which also transports bile acids, is very hepato-specific; a major 3.2 kb and several minor hybridizing bands were observed only in RNA from liver and no other tissue. The specific cell types that express this transporter were examined by *in situ* hybridization of OATP2 riboprobe to human liver samples. Strong hybridization signal was seen localized to hepatocytes throughout the liver lobule with no significant difference in signal intensity among centrilobular, midzonal or periportal regions. No signal was observed in bile ducts, Kupffer cells, or blood vessels, nor in any cell types from human lung (data not shown).

OATP-RP1 is expressed in nearly all tissues tested with highest abundance in skeletal muscle, lung, placenta, and heart. OATP-RP2 is ubiquitously expressed in all tissues tested. OATP-RP4 has a much more restricted pattern of expression with abundant transcipts in skeletal muscle and heart and much less in prostate and thymus. The expression of OATP-RP5 is likewise tissue specific, with brain and testes being the only sites where transcripts were detected.

### Example 3

### Expression of OATP2 in transfected cells

293EBNA cells (Invitrogen, Inc.), an HEK293 cell derivative, were transiently transfected with the OATP2 expression vector pCEP-OATP2, or the pCEP4 vector alone (MOCK) and the transport of [³H]-labeled substrates was determined 24 hours later. Figure 2A shows specific uptake of [³H]-pravastatin and [³H]-DHEAS. Figures 2B and 2C show the specific uptake of [³H]-taurocholate and [125I]-thyroid hormone (T4), repsectively. The uptake of radiolabeled substrate for 5 minutes into cells transfected with pCEP-OATP2 or empty vector (MOCK) was determined in the absence (solid bars) and presence (open bars) of excess unlabeled substrate. Thus, OATP2 is a liver specific human transporter of at least some HMG CoA reductase inhibitors, bile acids, adrenal steroids, and thyroid hormone.

## Claims

1. A nucleic acid sequence encoding all or a portion of the organic anion transport protein OATP2, said nucleic acid sequence being selected from the group consisting of:
(a) a nucleic acid sequence comprising the nucleic acid sequence shown in SEQ ID NO: 1;
(b) a nucleic acid sequence encoding all or a portion of the amino acid sequence shown in SEQ ID NO: 2, wherein the portion transports pravastatin into hepatocytes;
(c) a nucleic acid sequence comprising the coding region of (a);
(d) a nucleotide sequence comprising the nucleic acid sequence of any one of (a) to (c) that differs due to the degeneracy of the genetic code;
(e) a nucleic acid sequence comprising the sequence encoding OATP2 deposited with the American Type Culture collection under Accession Number 207213; or
(f) a nucleotide sequence comprising the complement of the nucleotide sequence of any one of (a), (c) or (e).

2. An expression vector comprising a nucleic acid sequence of claim 1 and an expression control sequence operatively linked to the nucleic acid molecule.

3. A host cell including in an expressible manner the nucleic acid sequence of claim 1 or the vector of claim 2.

4. A method of producing OATP2, said methods comprising the steps of:
(a) inserting a nucleic acid sequence according to claim 1, encoding said OATP2 protein, into an appropriate expression vector;
(b) introducing said expression vector into an appropriate transfection host cell;
(c) growing said transfected host cells in an appropriate culture media; and
(d) purifying the OATP2 protein from said culture media.

5. An OATP2 encoded by the nucleotide sequence of claim 1 or obtainable by the method of claim 4.

6. An antibody specific for the OATP2 of claim 5.

7. The antibody of claim 7 wherein said antibody is a monoclonal antibody.

8. A fusion protein comprising the OATP2 of claim 5, attached to a second polypeptide.

9. A method for identifying a ligand which is capable of binding to the OATP2 of claim 5, said method comprising the steps of:
(a) reacting said OATP2 with said ligand which potentially is capable of binding to the OATP2 under conditions which permit the formation of ligand-OATP2 complexes; and
(b) assaying for ligand-OATP2 complexes, for free ligand, or for non-complexed OATP2.

10. A method for identifying a substrate which is capable of being transported by the OATP2 of claim 5 said method comprising the steps of:
(a) reacting said OATP2 with said substrate which is potentially capable of being transported by the said OATP2 under conditions which permit the movement of said substrate across a membrane; and
(b) assaying for the movement of said substrate across the membrane.

11. A method for identifying a modulator which is capable of augmenting or inhibiting the transport of a substrate by the OATP2 of claim 5 said method comprising the steps of:
(a) reacting said OATP2 with said substrate and said modulator which potentially is capable of augmenting or inhibiting the transport of a substrate under conditions which permit the movement of said substrate across a membrane;
and
(b) measuring the augmentation or inhibition of transport of said compound by said modulator.

## Patentansprüche

1. Nucleinsäuresequenz, codierend das gesamte oder einen Anteil des Transportproteins für organische Anionen OATP2, wobei die Nucleinsäuresequenz aus:
(a) einer Nucleinsäuresequenz, umfassend die Nucleinsäuresequenz, angegeben in SEQ ID NO: 1;
(b) einer Nucleinsäuresequenz, codierend die gesamte oder einen Anteil der Aminosäuresequenz, angegeben in SEQ ID NO: 2, wobei der Anteil Pravastatin in Hepatozyten transportiert;
(c) einer Nucleinsäuresequenz, umfassend die Codierungsregion von (a);
(d) einer Nucleotidsequenz, umfassend die Nucleinsäuresequenz nach einem von (a) bis (c), die sich aufgrund der Degeneriertheit des genetischen Codes unterscheidet;
(e) einer Nucleinsäuresequenz, umfassend die Sequenz, codierend OATP2, hinterlegt bei der American Type Culture Collection unter der Zugangsnummer 207213; oder
(f) einer Nucleotidsequenz, umfassend das Komplement der Nucleotidsequenz nach einem von (a), (c) oder (e), ausgewählt ist.

2. Expressionsvektor, umfassend eine Nucleinsäuresequenz nach Anspruch 1 und eine Expressionskontrollsequenz, operativ verknüpft mit dem Nucleinsäuremolekül.

3. Wirtszelle, einschließend in einer exprimierbaren Weise die Nucleinsäuresequenz nach Anspruch 1 oder den Vektor nach Anspruch 2.

4. Verfahren zum Erzeugen von OATP2, wobei die Verfahren die Schritte umfassen:
(a) Insertieren einer Nucleinsäuresequenz gemäß Anspruch 1, codierend das OATP2-Protein, in einen passenden Expressionsvektor;
(b) Einführen des Expressionsvektors in eine passende Transfektions-Wirtszelle;
(c) Züchten der transfizierten Wirtszellen in einem passenden Kulturmedium; und
(d) Reinigen des OATP2-Proteins von dem Kulturmedium.

5. OATP2, codiert durch die Nucleotidsequenz nach Anspruch 1 oder erhältlich durch das Verfahren nach Anspruch 4.

6. Antikörper, spezifisch für das OATP2 nach Anspruch 5.

7. Antikörper nach Anspruch 6, wobei der Antikörper ein monoklonaler Antikörper ist.

8. Fusionsprotein, umfassend das OATP2 nach Anspruch 5, verbunden mit einem zweiten Polypeptid.

9. Verfahren zum Identifizieren eines Liganden, welcher imstande ist, an das OATP2 nach Anspruch 5 zu binden, wobei das Verfahren die Schritte umfaßt:
(a) Umsetzen des OATP2 mit dem Liganden, welcher potentiell imstande ist, an das OATP2 zu binden, unter Bedingungen, welche die Bildung von Ligand-OATP2-Komplexen gestatten; und
(b) Durchführen von Assays für Ligand-OATP2-Komplexe, für freien Liganden oder für nicht komplex gebundenes OATP2.

10. Verfahren zum Identifizieren eines Substrats, welches imstande ist, durch das OATP2 nach Anspruch 5 transportiert zu werden, wobei das Verfahren die Schritte umfaßt:
(a) Umsetzen des OATP2 mit dem Substrat, welches potentiell imstande ist, durch das OATP2 transportiert zu werden, unter Bedingungen, welche die Bewegung des Substrats durch eine Membran gestatten; und
(b) Durchführen von Assays für die Bewegung des Substrats durch die Membran.

11. Verfahren zum Identifizieren eines Modulators, welcher imstande ist, den Transport eines Substrats durch das OATP2 nach Anspruch 5 zu verstärken oder zu hemmen, wobei das Verfahren die Schritte umfaßt:
(a) Umsetzen des OATP2 mit dem Substrat und dem Modulator, welcher potentiell imstande ist, den Transport eines Substrats zu verstärken oder zu hemmen, unter Bedingungen, welche die Bewegung des Substrats durch eine Membran gestatten; und
(b) Messen der Verstärkung oder Hemmung des Transports der Verbindung durch den Modulator.

## Revendications

1. Séquence d'acide nucléique codant toute ou une partie de la protéine de transport d'anion organique OATP2, ladite séquence d'acide nucléique étant sélectionnée parmi le groupe consistant en:
(a) une séquence d'acide nucléique comprenant la séquence d'acide nucléique présentée dans la SEQ ID NO:1;
(b) une séquence d'acide nucléique codant toute ou une partie de la séquence d'acides aminés présentée dans la SEQ ID NO:2, où la partie transporte la pravastatine dans les hépatocytes;
(c) une séquence d'acide nucléique comprenant la région codante de (a);
(d) une séquence de nucléotides comprenant la séquence d'acide nucléique de l'un quelconque de (a) à (c) qui diffère à cause de la dégénérescence du code génétique;
(e) une séquence d'acide nucléique comprenant la séquence codant OATP2 déposée auprès de la American Type Culture Collection sous le numéro d'accès 207213; ou
(f) une séquence de nucléotides comprenant le complément de la séquence de nucléotides de l'un quelconque de (a), (c) ou (e).

2. Vecteur d'expression comprenant une séquence d'acide nucléique de la revendication 1 et une séquence de contrôle d'expression liée de manière opérationnelle à la molécule d'acide nucléique.

3. Cellule hôte incluant d'une manière exprimable la séquence d'acide nucléique de la revendication 1 ou le vecteur de la revendication 2.

4. Procédé de production de l'OATP2, ledit procédé comprenant les étapes consistant à:
(a) insérer une séquence d'acide nucléique selon la revendication 1 codant ladite protéine OATP2, dans un vecteur d'expression approprié;
(b) introduire ledit vecteur d'expression dans une cellule hôte de transfection appropriée;
(c) cultiver lesdites cellules hôtes transfectées dans un milieu de culture approprié; et
(d) purifier la protéine OATP2 dudit milieu de culture.

5. OATP2 codée par la séquence de nucléotides de la revendication 1 ou pouvant être obtenue par le procédé de la revendication 4.

6. Anticorps spécifique pour l'OATP2 de la revendication 5.

7. L'anticorps de la revendication 6, où ledit anticorps est un anticorps monoclonal.

8. Protéine de fusion comprenant l'OATP2 de la revendication 5, attachée à un deuxième polypeptide.

9. Procédé d'identification d'un ligand qui est capable de se lier à l'OATP2 de la revendication 5, ledit procédé comprenant les étapes consistant à:
(a) mettre ladite OATP2 à réagir avec ledit ligand qui est potentiellement capable de se lier à l'OATP2, dans des conditions qui permettent la formation de complexes de ligand-OATP2; et
(b) analyser les complexes de ligand-OATP2 pour un ligand libre ou une OATP2 non complexée.

10. Procédé d'identification d'un substrat qui est capable d'être transporté par l'OATP2 de la revendication 5, ledit procédé comprenant les étapes consistant à:
(a) mettre ladite OATP2 à réagir avec ledit substrat qui est potentiellement capable d'être transporté par ladite OATP2, dans des conditions qui permettent le mouvement dudit substrat à travers une membrane; et
(b) analyser pour le mouvement dudit substrat à travers la membrane.

11. Procédé d'identification d'un modulateur qui est capable d'augmenter ou d'inhiber le transport d'un substrat par l'OATP2 de la revendication 5, ledit procédé comprenant les étapes consistant à:
(a) mettre ladite OATP2 à réagir avec ledit substrat et ledit modulateur qui est potentiellement capable d'augmenter ou d'inhiber le transport d'un substrat, dans des conditions qui permettent le mouvement dudit substrat à travers une membrane; et
(b) mesurer l'augmentation ou l'inhibition du transport dudit composé par ledit modulateur.
